# EUROPÄISCHE PATENTSCHRIFT

(11) **EP 3 045 133 B1**
(45) Veröffentlichungstag und Bekanntmachung des Hinweises auf die Patenterteilung: **27.03.2019**
(21) Anmeldenummer: 15202438.6
(22) Anmeldetag: 23.12.2015
(51) Int. Cl.: A61B 18/08, A61B 17/3211, A61B 18/00, A61B 17/00

(54) **KLINGE FÜR EIN SCHNEIDEINSTRUMENT, SKALPELLHALTER FÜR EINE KLINGE SOWIE VERFAHREN ZUM HERSTELLEN EINER KLINGE**
BLADE FOR A CUTTING INSTRUMENT, SCAPEL HOLDER FOR A CUTTING BLADE AND METHOD FOR PRODUCING A BLADE
LAME POUR UN INSTRUMENT DE DECOUPE, PORTE-SCALPEL POUR UNE LAME ET PROCEDE DE FABRICATION D'UNE LAME

(30) Priorität: 13.01.2015 DE 102015200308
(43) Veröffentlichungstag der Anmeldung: 20.07.2016
(73) Patentinhaber: ROBERT BOSCH GMBH, 70442 Stuttgart (DE)
(72) Erfinder: Sedlmayr, Andreas, 75173 Pforzheim (DE); Glanz, Uwe, 71679 Asperg (DE); Heeren, Imke, 70199 Stuttgart (DE); Schellenberg, Inga, 76137 Karlsruhe (DE); Loibl, Thomas, 87561 Oberstdorf (DE)

(56) Entgegenhaltungen:
- WO-A1-93/21838
- WO-A1-94/09714
- WO-A2-2008/094564
- DE-A1- 4 407 797

## Beschreibung

### Stand der Technik

Die vorliegende Erfindung bezieht sich auf eine Klinge für ein Schneideinstrument, insbesondere für einen medizinischen Einsatz, einen entsprechenden Skalpellhalter für eine Klinge sowie ein Verfahren zum Herstellen einer entsprechenden Klinge.

Bei medizinischen Eingriffen werden Schnitte durchgeführt, deren Schnittkanten verödet werden, um den Blutfluss zu stoppen. Das Veröden kann im Idealfall instantan erfolgen, um Blutverluste zu minimieren und die Sichtbarkeit des Arbeitsbereichs für den Arzt zu verbessern. Derzeit werden beispielsweise Elektroskalpelle verwendet. Diese funktionieren wie folgt: Ein Strom wird durch den menschlichen Körper geleitet und führt durch thermische Erwärmung zur Verödung durch Koagulation der Eiweiße, wodurch Adern und Gewebeteile verschweißt werden. Bei Elektroskalpellen wird zwischen der monopolaren Technik und der bipolaren Technik unterschieden.

Bei der monopolaren Technik (Hauptanwendung) wird ein Pol der Spannungsquelle über eine möglichst große Fläche mit dem Patienten verbunden. Das Messer bildet den zweiten Pol. Hierbei kann es passieren, dass der Strom nicht zielgenau an der zu verödenden Stelle fließt, sondern auch noch einen anderen Weg durch den Körper oder über die Körperoberfläche des Patienten wählt und es dadurch zu heftigen Verbrennungen an falscher Stelle kommt.

Bei der bipolaren Technik sind beide Pole am Werkzeug beispielsweise Pinzette, oder Zange angeordnet. Hier ist man auf zweipolige Werkzeugkonstruktionen wie Schere oder Zange angewiesen. Die dennoch auftretende Flüssigkeitsbildung (Blut, Gewebeflüssigkeit...) nimmt die Sicht und wird über ein zusätzliches Absaugwerkzeug entfernt.

Aus WO 1993/21938 A1 und DE 44 07 797 A1 sind chirurgische Klingen mit einer Heizeinrichtung bekannt.

### Offenbarung der Erfindung

Vor diesem Hintergrund werden mit dem hier vorgestellten Ansatz eine Klinge für ein Schneideinstrument, insbesondere für einen medizinischen Einsatz, ein entsprechender Skalpellhalter für eine Klinge sowie ein Verfahren zum Herstellen einer Klinge gemäß den Hauptansprüchen vorgestellt. Vorteilhafte Ausgestaltungen ergeben sich aus den jeweiligen Unteransprüchen und der nachfolgenden Beschreibung. Alle im Folgenden genannten Beispiele und Ausführungen, die nicht unter die unabhängigen Ansprüche fallen, sind nicht Teil der Erfindung.

Es wird ein keramisches Messer mit innenliegendem Heizer vorgestellt, das vor oder während des Schneidprozesses beheizt werden kann. Auf diesem Weg dann die Koagulation zeitgleich mit dem Schneiden (Resektion) erfolgen, ohne dass es zu Austritt ungewollter Blutmengen kommt. Optional kann zusätzlich eine Möglichkeit einer Flüssigkeitsabsaugung als auch Flüssigkeitsdosierung, bzw. Flüssigkeitseinleitung zur Wirkstoffabgabe integriert werden. Ist ein Kanal auf der Höhe des Messerrückens mit der Öffnung Richtung Messerspitze hin angebracht, könnte beispielsweise auch Luft oder ein anderes Gas oder therapeutisches Aerosol einblasen werden.

Es wird eine Klinge für ein Schneideinstrument vorgestellt, insbesondere eine Klinge für einen medizinischen Einsatz, gekennzeichnet durch eine erste Keramikschicht, eine zweite Keramikschicht und eine Heizeinrichtung, die zwischen der ersten Keramikschicht und der zweiten Keramikschicht angeordnet ist.

Unter einem Schneideinstrument kann ein Skalpell oder ein chirurgisches Instrument zum Durchtrennen von Gewebe verstanden werden. Unter der Klinge kann eine Klinge eines beheizbaren Skalpells zur instantanen Koagulation verstanden werden. So kann es sich bei dem Schneideinstrument um ein feines Messer mit sehr scharfer Klinge handeln. Die Klinge kann eine Schneide, zwei an die Schneide angrenzende und sich gegenüberliegende Klingenseiten, einen Klingenrücken und einen Schaft aufweisen. Dabei kann die Heizeinrichtung zwischen den beiden Klingenseiten angeordnet sein. Die Heizeinrichtung kann im Bereich der Schneide angeordnet sein. In einer Ausführungsform kann die Klinge auch mehr als eine Schneide aufweisen. Vorteilhaft ist die Klinge biokompatibel, insbesondere im Vergleich zu einer Metallklinge kann die hier beschriebene Klinge in höchstem Maße biokompatibel sein. Die Klinge kann gegenüber einem Metallskalpell eine höhere Standzeit aufweisen und somit über einen langen oder im Vergleich zum Metallskalpell längeren Nutzungszeitraum scharf bleiben. Vorteilhaft fließt kein Strom durch einen menschlichen Körper bei einem medizinischen Einsatz zum Durchtrennen von Gewebe.

Erfindungsgemäß sind die erste Keramikschicht und die zweite Keramikschicht zu einem monolithischen Bauteil verbunden. So kann eine hohe Standfestigkeit erzielt werden.

Die Heizeinrichtung kann zumindest einen Anschluss aufweisen, der gegenüber der zweiten Keramikschicht freiliegt. So kann die Heizeinrichtung einfach elektrisch kontaktierbar sein. So kann der Anschluss zumindest ein Kontaktpad umfassen. In einer günstigen Ausführungsform kann die Heizeinrichtung zumindest zwei Kontaktpads zum elektrischen Kontaktieren aufweisen, die von der zweiten Schicht unbedeckt sind.

Ferner können die erste Keramikschicht und ergänzend oder alternativ die zweite Keramikschicht zumindest teilweise aus einer keramischen Grünfolie gefertigt sein. Die erste Keramikschicht und ergänzend oder alternativ die zweite Keramikschicht können Zirkonoxid aufweisen.

Die Heizeinrichtung kann eine Temperaturmesseinrichtung umfassen. So kann eine Temperatur der Klinge geregelt werden. Ferner kann die Heizeinrichtung einen Heizdraht aufweisen, um die Klinge mehrfach auf über 500°C aufzuheizen. Insbesondere kann eine Materialdicke des Heizdrahts oder ein Material des Heizdrahts geeignet sein, die Klinge mehrfach auf über 500°C aufzuheizen. Der Heizdraht kann als ein Heizmäander ausgebildet sein.

Erfindungsgemäß ist zwischen der ersten Keramikschicht und der zweiten Keramikschicht zumindest ein Fluidkanal ausgebildet. Unter einem Fluidkanal kann ein Kanal, ein Rohr oder eine Kavität verstanden werden. In einer besonderen Ausbildungsform kann die Klinge zwei Fluidkanäle oder mehr als zwei Fluidkanäle aufweisen. Der Fluidkanal kann dabei ausgebildet sein, ein Fluid wie eine Körperflüssigkeit oder ein Medikament, ein Spülmedium oder therapeutisches Fluid zu transportieren. So kann die Klinge zumindest einen Sammelkanal aufweisen, von dem eine Mehrzahl von Fluidkanälen abzweigen. Die integrierten Kavitäten in Form von Fluidkanälen können vorteilhaft eine gezielte Absaugung von Flüssigkeiten oder aber auch ein Einleiten von Spülmedium oder Medikamenten ermöglichen.

Der zumindest eine Fluidkanal kann zumindest eine Öffnung als Einlass und ergänzend oder alternativ einen Auslass für ein Fluid im Bereich einer Schneide und ergänzend oder alternativ einer Klingenseite und ergänzend oder alternativ einem Klingenrücken und ergänzend oder alternativ einem Schaft der Klinge aufweisen. Vorteilhaft kann über die zumindest eine Öffnung des zumindest einen Fluidkanals eine Absaugung von Flüssigkeiten oder aber auch ein Einleiten von Spülmedium oder Medikamenten ermöglicht werden.

Ein Querschnitt des Fluidkanals kann einen Durchmesser in einem Toleranzbereich zwischen 0,20 und 3 mm aufweisen. So kann der Querschnitt an eine Viskosität und ein Strömungsverhalten eines zu leitenden Fluids angepasst werden. Der Querschnitt des Fluidkanals kann an eine Geometrie der Klinge angepasst sein.

Der Querschnitt des Fluidkanals kann zumindest abschnittsweise kreisrund, ellipsoid oder schlitzförmig ausgeformt sein. So kann ein erster Abschnitt kreisrund ausgeformt und ein zweiter Abschnitt beispielsweise ellipsoid oder schlitzförmig ausgeformt sein. So können unterschiedliche Abschnitte des Fluidkanals verschieden voneinander ausgeformt sein. Die zumindest eine Öffnung des Fluidkanals kann kreisrund, ellipsoid oder schlitzförmig ausgeformt sein. Eine zweite Öffnung kann verschieden zu einer ersten Öffnung ausgeformt sein. So können die Öffnung und der Querschnitt des Fluidkanals an eine Viskosität und ein Strömungsverhalten eines zu leitenden Fluids angepasst werden.

Eine Ebene, in der die Heizeinrichtung angeordnet ist, kann durch eine Schneide der Klinge verlaufen. Insbesondere kann die Klinge symmetrisch zu der Ebene ausgeformt sein, die durch die Schneide verläuft. Die Klinge kann mit einem standardisierten Skalpellhalter kombinierbar sein.

Es wird ein Skalpellhalter für eine Variante einer hier vorgestellten Klinge für ein Schneideinstrument vorgestellt, wobei der Skalpellhalter zumindest einen Anschluss zum Kontaktieren der Heizeinrichtung der Klinge aufweist.

Der Skalpellhalter kann einen Fluidanschluss zur Fluidzuführung und/oder Fluidabführung für den Fluidkanal der Klinge aufweisen. Der Fluidanschluss kann mit einer Öffnung des Fluidkanals koppelbar sein. So kann der Fluidanschluss zu einer Öffnung des Fluidkanals der Klinge ausrichtbar sein.

Der Skalpellhalter kann einen Energiespeicher, insbesondere zum Betreiben der Heizeinrichtung, aufweisen. Ferner kann der Skalpellhalter zumindest einen Tank für ein Fluid aufweisen. Der Tank kann über eine Pumpe mit dem Fluidanschluss verbunden sein.

Es wird ein Verfahren zum Herstellen einer Klinge für ein Schneideinstrument vorgestellt, wobei das Verfahren die folgenden Schritte umfasst:
Anordnen einer ersten Keramik-bildenden Lage zum Erzeugen einer ersten Keramikschicht;
Anordnen einer Heizeinrichtung auf der ersten Keramik-bildenden Lage und/oder der ersten Keramikschicht;
Anordnen einer zweiten Keramik-bildenden Lage zum Erzeugen einer zweiten Keramikschicht auf der ersten Keramik-bildenden Lage, sodass die Heizeinrichtung zwischen der ersten Keramik-bildenden Lage und der zweiten Keramik-bildenden Lage angeordnet ist;
Temperieren der Anordnung aus erster und zweiter Keramik-bildender Lage mit der Heizeinrichtung, wobei die erste Keramikschicht und die zweite Keramikschicht die Heizeinrichtung umschließen; und
Schleifen der gesinterten Anordnung, um die Klinge für das Schneideinstrument oder ein chirurgisches Instrument zu erhalten.

Unter einer Keramik-bildenden Lage kann beispielsweise eine Grünfolie oder eine Schicht aus Keramik-Spritzguss verstanden werden. Unter dem Schritt des Temperierens kann ein Sintern oder Entbindern verstanden werden. Dabei kann die Anordnung beispielsweise über 1200 °C erhitzt werden. Im Schritt des Temperierens können die Keramik-bildenden Lagen in die Keramikschichten gewandelt werden.

Die hier beschriebenen Keramik-bildenden Lage können beispielsweise als Grünfolien über einen Schlickerguss hergestellt werden. Sie sind "Endlosware" die später durch einen Schneidprozess vereinzelt werden. Alternativ könnte man die Keramik-bildenden Lagen auch über Keramikspritzguss als gezielte Formteile herstellen und diese dann mit den Heizmäandern bedrucken und zwei passende Teile zusammenlaminieren oder nach dem Bedrucken in einem wiederholten Spritzvorgang überspritzen. Bei diesem Verfahren hätte man sehr hohe Designfreiheit.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Ein Aspekt der vorliegenden Erfindung ist die Realisierung eines beheizbaren, keramischen Messers über die keramische Mehrschichttechnologie. Die keramische Mehrschichttechnologie wird beispielsweise in der Fertigung keramischer Abgassensoren (beispielsweise Lambdasonde) genutzt.

Vorteilhaft kann eine direkte Beheizung der inerten, keramischen Klinge über den innenliegenden Heizer erfolgen, ohne dass hierbei Heizermaterial in direktem Kontakt mit dem Körper eines Patienten steht. Dabei ist vorteilhaft das keramische Klingenmaterial im höchsten Maße biokompatibel. Von Vorteil ist eine höhere Standzeit des Materials gegenüber Metallskalpellen, da die Klinge über einen langen Nutzungszeitraum scharf bleibt. Eine Sterilisierung über Eigentemperatur des Messers kann erzielt werden. So kann eine Temperaturerhöhung über 500°C ermöglicht werden und somit eine Säuberung des Messers von anhaftenden Geweberesten o.ä. durch Pyrolyse erzielt werden. Vorteilhaft fließt kein Strom durch den Körper - keine Kollateralschäden während einer Operation. Optional ermöglichen die integrierten Kavitäten in Form von Kanälen eine Absaugung von Flüssigkeiten oder aber auch ein Einleiten von Spülmedium oder Medikamenten.

Der hier vorgestellte Ansatz wird nachstehend anhand der beigefügten Zeichnungen beispielhaft näher erläutert. Es zeigen:
Fig. 1 eine vereinfachte Darstellung einer Klinge gemäß einem Ausführungsbeispiel der vorliegenden Erfindung;
Fig. 2 eine vereinfachte Darstellung der Keramikschichten und der Heizeinrichtung gemäß einem Ausführungsbeispiel der vorliegenden Erfindung;
Fig. 3 eine vereinfachte Darstellung einer ersten Keramikschicht gemäß einem Ausführungsbeispiel der vorliegenden Erfindung;
Fig. 4 eine vereinfachte Darstellung einer zweiten Keramikschicht gemäß einem Ausführungsbeispiel der vorliegenden Erfindung;
Fig. 5 eine vereinfachte Darstellung einer Klinge gemäß einem Ausführungsbeispiel der vorliegenden Erfindung;
Fig. 6 eine vereinfachte Darstellung von Formen der Schneide gemäß einem Ausführungsbeispiel der vorliegenden Erfindung;
Fig. 7 eine vereinfachte Darstellung eines Schnellverschlusses der Klinge gemäß einem Ausführungsbeispiel der vorliegenden Erfindung;
Fig. 8 eine vereinfachte Darstellung eines Schneideinstruments mit einer Klinge und einem Skalpellhalter gemäß einem Ausführungsbeispiel der vorliegenden Erfindung; und
Fig. 9 ein Ablaufdiagramm eines Verfahrens gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

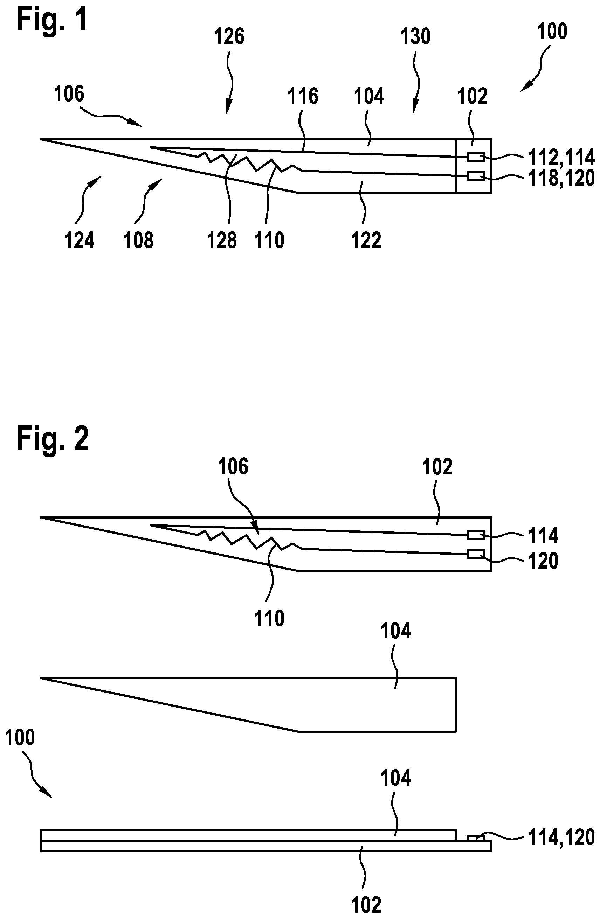

Fig. 1 zeigt eine vereinfachte Darstellung einer Klinge 100 gemäß einem Ausführungsbeispiel der vorliegenden Erfindung. Die Klinge 100 weist eine erste Keramikschicht 102, eine zweite Keramikschicht 104 sowie eine zwischen der ersten Keramikschicht 102 und der zweiten Keramikschicht 104 angeordnete Heizeinrichtung 106 auf.

Die Heizeinrichtung 106 umfasst als ein Heizelement 108 in dem dargestellten Ausführungsbeispiel ein Heizmäander 110, ein erstes Kontaktpad 112 als ersten Anschluss 114, eine erste Zuleitung 116 zwischen dem ersten Kontaktpad 112 und dem Heizmäander 110, ein zweites Kontaktpad 118 als zweiten Anschluss 120 sowie eine zweite Zuleitung 122 zwischen dem zweiten Kontaktpad 118 und dem Heizmäander 110. Die zweite Keramikschicht 104 ist kleiner als die erste Keramikschicht 102, sodass die Anschlüsse 114, 120 freiliegen und einfach kontaktierbar sind. Unter dem Heizmäander 110 kann ein Heizdraht, ein Heizermäander oder ein Temperaturmäander verstanden werden.

In einem besonderen Ausführungsbeispiel umfasst die Heizeinrichtung 106 ein Heizelement 108 aus Platin mit einem hochohmigen Heizmäander 110 und niederohmigen Zuleitungen 116, 122. Der hochohmige Heizmäander 110 besteht beispielsweise aus einer Platinlegierung mit Palladium oder Rhodium.

In einem Ausführungsbeispiel sind die erste Keramikschicht 102 und die zweite Keramikschicht 104 zu einem monolithischen Bauteil beziehungsweise einem monolithischen Keramikkörper verbunden. Der Keramikkörper der Klinge 100 weist in einem Ausführungsbeispiel einen schichtartigen Aufbau auf, wobei der Heizmäander 110 und die Zuleitungen 116, 122 mittels Siebdrucktechnik auf einzelne Keramikfolien aufgebracht werden.

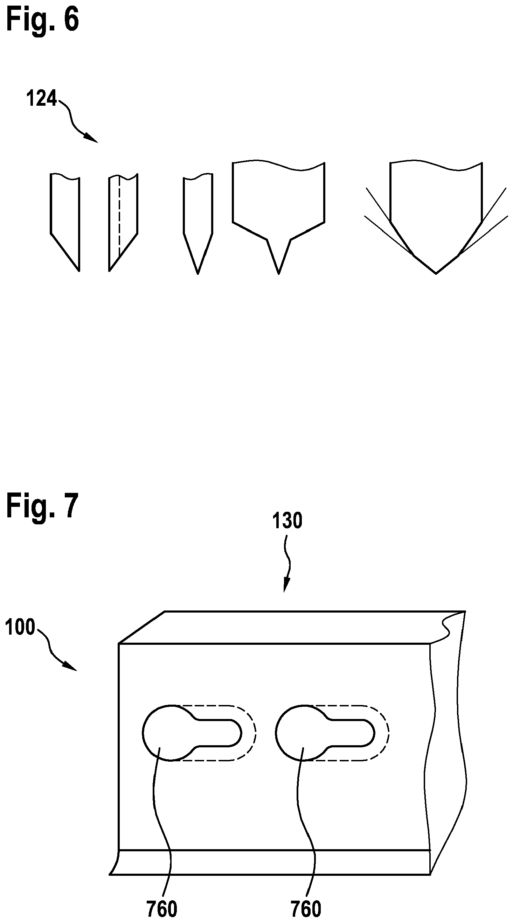

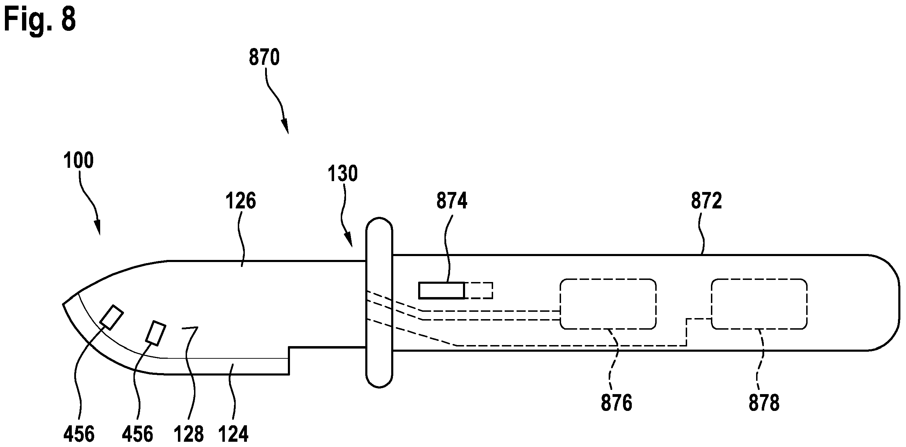

Die Klinge weist eine Schneide 124, einen Klingenrücken 126, eine Klingenseite 128 sowie einen Schaft 130 auf. Die Form der Schneide 124 wird in Fig. 6 detaillierter beschrieben. So handelt es sich beispielsweise in jeweils einem besonderen Ausführungsbeispiel um eine Clip-Point-Klinge oder eine Drop-Point-Klinge. Der Schaft 130 kann ausgebildet sein, mit einem Skalpellhalter verbunden zu werden, wie dies beispielsweise in Fig. 7 und Fig. 8 detaillierter beschrieben ist.

Fig. 2 zeigt eine vereinfachte Darstellung der Keramikschichten 102, 104 und der Heizeinrichtung 106 gemäß einem Ausführungsbeispiel der vorliegenden Erfindung. Bei den Keramikschichten 102, 104 und der Heizeinrichtung 106 sowie der Klinge 100 kann es sich um eine Variante eines in Fig. 1 dargestellten Ausführungsbeispiels einer Klinge 100, der Keramikschichten 102, 104 sowie der Heizeinrichtung 106 handeln.

Fig. 2 zeigt in drei zusammengehörenden Darstellungen die Teile einer Klinge 100. Zuoberst ist in Fig. 2 die erste Keramikschicht 102 mit darauf angeordneter Heizeinrichtung 106 dargestellt. Dabei weist die Heizeinrichtung 106 zwei Anschlüsse 114, 120 auf. Die Heizeinrichtung 106 umfasst im Bereich der Schneide 124 einen Heizmäander 110. Die mittlere Abbildung zeigt in Fig. 2 die zweite Keramikschicht 104. Die untere Abbildung in Fig. 2 zeigt einen Schnitt quer zu den dargestellten Keramikschichten 102, 104, sodass die freiliegenden Anschlüsse 114, 120 zu erkennen sind.

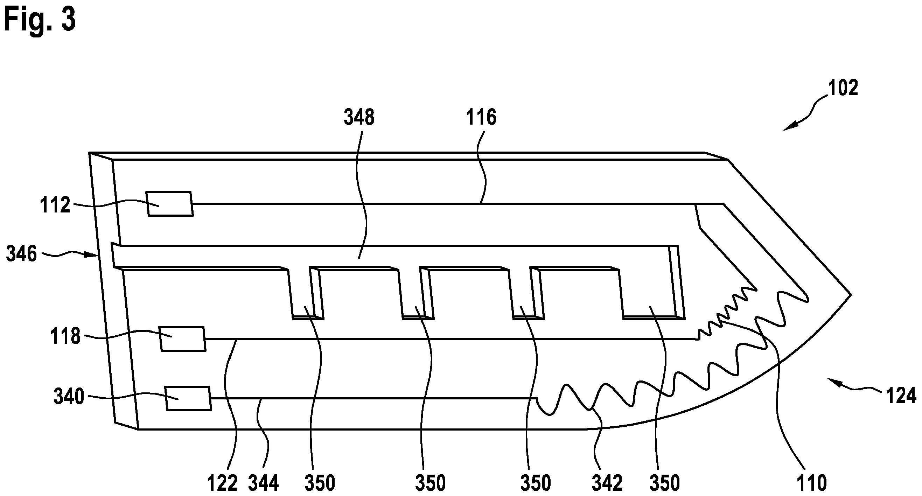

Fig. 3 zeigt eine vereinfachte dreidimensionale Darstellung einer ersten Keramikschicht 102 gemäß einem Ausführungsbeispiel der vorliegenden Erfindung. Bei der ersten Keramikschicht 102 kann es sich um eine Variante einer in den vorangegangenen Figuren gezeigten Ausführungsform einer ersten Keramikschicht 102 handeln. An einer Oberfläche der ersten Keramikschicht 102 ist eine Heizeinrichtung 102 angeordnet. Die Heizeinrichtung 106 umfasst ein erstes Kontaktpad 112, ein zweites Kontaktpad 118 sowie ein drittes Kontaktpad 340, einen ersten Heizmäander 110 sowie einen zweiten Heizmäander 342, eine erste Zuleitung 116 zwischen dem ersten Kontaktpad 108 und dem ersten und zweiten Heizmäandern 110, 342, eine zweite Zuleitung 122 zwischen dem zweiten Kontaktpad 118 und dem ersten Heizmäander 110 und eine dritte Zuleitung 344 zwischen dem dritten Kontaktpad 340 und dem zweiten Heizmäander 342.

Je nach Ausführungsbeispiel weisen die Zuleitungen 116, 122, 344 eine Breite zwischen 100 µm und 400 µm und eine Dicke zwischen 5 µm und 20 µm auf. Je nach Ausführungsbeispiel weisen die Heizmäander 110, 342 eine Breite zwischen 40 µm und 100 µm und eine Dicke zwischen 2 µm und 20 µm auf.

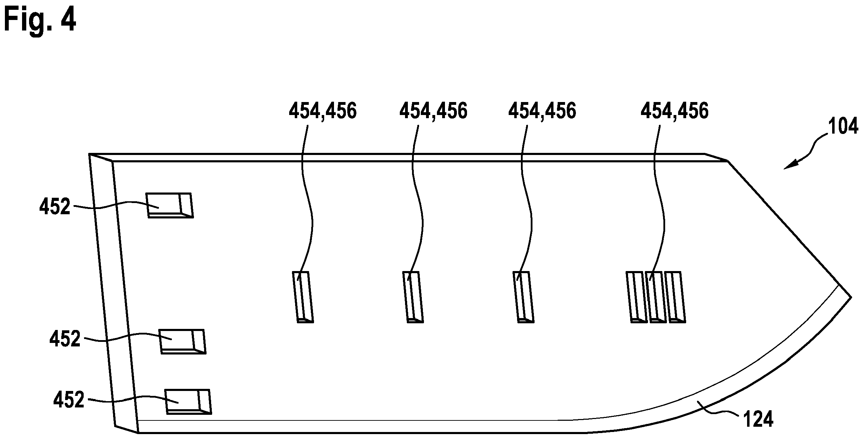

In der ersten Keramikschicht 102 ist ein Fluidkanal 346 angeordnet. Ein Sammelkanal 348 des Fluidkanals 346 verläuft im Wesentlichen entlang einer Haupterstreckungsrichtung der ersten Keramikschicht 102. Von dem Sammelkanal 348 zweigen im Wesentlichen quer zu dem Sammelkanal 348 vier Dosierkanäle 350 ab in Richtung der Schneide 124 der Klinge, das heißt in der Darstellung nach unten. Die Dosierkanäle 350 sind quasi als Sackgassen ausgeformt, wobei diese zu in der folgenden Fig. 4 dargestellten Öffnungen in der zweiten Keramikschicht führen. Die Anzahl der Kanäle ist jedoch nicht fix, es kann je nach Anforderung die Anzahl variiert oder auch ihre Position verändert werden.

In einem Ausführungsbeispiel handelt es sich bei dem Fluidkanal 346 um einen Absaugkanal 346.

Der Fluidkanal 346 weist in einem Ausführungsbeispiel eine Höhe zwischen 0,1 mm und 3 mm und eine Tiefe zwischen 0,1 mm und 0,8 mm auf.

In einem Ausführungsbeispiel ist der erste Heizmäander 110 als ein Temperaturmäander ausgeführt.

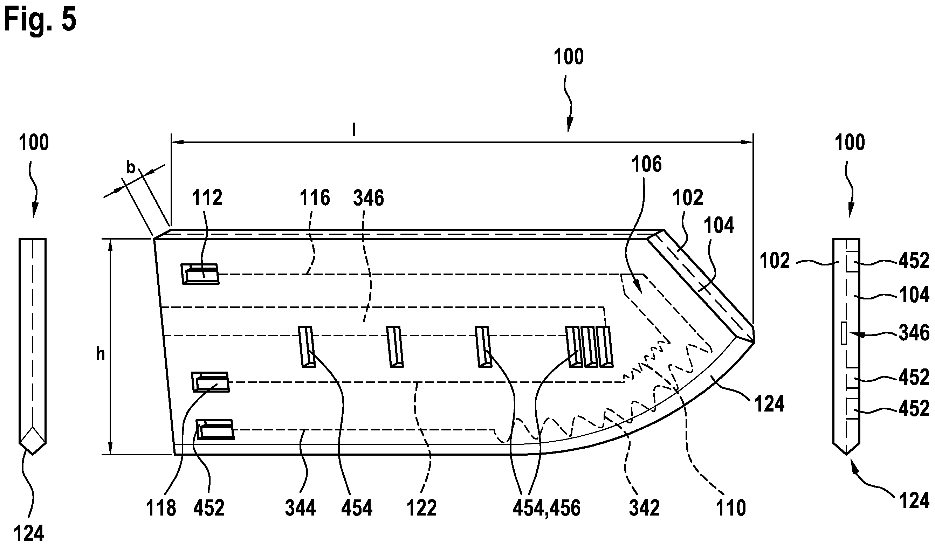

Fig. 4 zeigt eine vereinfachte Darstellung einer zweiten Keramikschicht 104 gemäß einem Ausführungsbeispiel der vorliegenden Erfindung. Bei der zweiten Keramikschicht 104 kann es sich um eine Variante einer in den Figuren Fig. 1 und Fig. 2 gezeigten Ausführungsbeispielen einer zweiten Keramikschicht 104 handeln. Wie in der folgenden Fig. 5 dargestellt, ist die zweite Keramikschicht 104 mit der in Fig. 3 dargestellten Keramikschicht 102 zu einer Klinge 100 kombinierbar.

Die zweite Keramikschicht 104 weist eine Mehrzahl von Durchführungen 452, 454 auf. Drei Durchführungen 452 sind derart positioniert, dass die auf der in Fig. 3 dargestellten ersten Keramikschicht 102 angeordneten Kontaktpads 112, 118, 340 beziehungsweise Anschlüsse 114, 120 freiliegen, wenn die beiden Keramikschichten 102, 104 zusammengefügt werden, wie in Fig. 5 dargestellt. Weiterhin sind Durchführungen 454 als Öffnungen 456 als Einlass oder Auslass für ein Fluid im Bereich der Klingenseite positioniert. Die Durchführungen 454 sind passend zu den in Fig. 3 dargestellten Dosierkanälen 350 angeordnet. Aufgrund der schlitzförmigen Ausformung der Öffnungen 456 werden diese auch als Haifischkiemen bezeichnet. Einem Dosierkanal sind dabei drei Öffnungen 456 zugeordnet, den anderen Dosierkanälen jeweils eine Öffnung 456.

Die Durchführungen 452 weisen in einem Ausführungsbeispiel eine Länge zwischen 1 mm und 4 mm und eine Breite zwischen 0,5 mm und 2 mm auf. Die Öffnungen 456 weisen in einem alternativen Ausführungsbeispiel eine Länge zwischen 0,1 mm und 3,5 mm und eine Breite zwischen 0,1 mm und 3,5 mm auf.

Fig. 5 zeigt eine vereinfachte Darstellung einer Klinge 100 gemäß einem Ausführungsbeispiel der vorliegenden Erfindung. Bei der Klinge 100 kann es sich um eine Variante eines in Fig. 1 und Fig. 2 gezeigten Ausführungsbeispiels einer Klinge 100 handeln. Die Klinge 100 umfasst eine erste Keramikschicht 102 und eine zweite Keramikschicht 104 sowie eine zwischen den Keramikschichten angeordnete Heizeinrichtung 106. Bei den Keramikschichten 102, 104 handelt es sich um die in Fig. 3 dargestellte erste Keramikschicht 102 und die in Fig. 4 dargestellte zweite Keramikschicht 104. Die Klinge 100 ist links in einer Aufsicht, in der Mitte in einer Seitenansicht und rechts in einer Rückansicht dargestellt. Die links dargestellte Aufsicht zeigt insbesondere eine Form der Schneide 124 der Klinge 100. Varianten der Schneide 124 sind beispielhaft in Fig. 6 dargestellt.

In der mittleren Seitenansicht der Klinge 100 ist die zweite Keramikschicht 104 mit den Durchführungen 452, 454 vor der ersten Keramikschicht 102 angeordnet und dargestellt. Zwischen der ersten Keramikschicht 102 und der zweiten Keramikschicht 104 ist eine Heizeinrichtung 106 angeordnet. Ein Fluidkanal 346 in der ersten Keramikschicht 102 ist mit den Öffnungen 456 in der zweiten Keramikschicht 104 verbunden. Die Durchführungen 452 sind derart angeordnet, dass die Kontaktpads 112, 118, 340 freiliegen und kontaktierbar sind.

Die Abmessungen der Klinge 100 variieren in unterschiedlichen Ausführungsbeispielen. Dabei weist die Klinge beispielsweise eine Länge I zwischen 15 mm und 50 mm, eine Breite b zwischen 0,25 mm und 1 mm sowie eine Höhe h zwischen 5 mm und 20 mm auf.

Die rechts dargestellte Rückansicht zeigt einen Anschluss für den Fluidkanal 346 sowie die Durchführungen 452 in der zweiten Keramikschicht 104.

Die Tiefe der Durchführungen 452 reicht bis zu den Kontaktpads 112, 118, 340 beziehungsweise den entsprechenden Anschlüssen.

Die in Fig. 3 bis Fig. 5 genannten Abmessungen sind unter anderem von der Messergeometrie beziehungsweise der Geometrie und den Abmessungen der Klinge 100 abhängig.

Das beheizbare Messer kennzeichnet sich durch die Einbettung eines Heizmäanders und eines Temperaturmäanders (beispielsweise Platin) zwischen zwei keramischen Grünfolien (beispielsweise Zirkonoxid). Hierbei werden beispielsweise der Heizmäander und die Anschlusskontakte über Siebdruck auf eine keramische Grünfolie (ungesintert) appliziert.

In einem Ausführungsbeispiel kann nach einem Trocknungsschritt eine zweite Grünfolie aufgelegt werden und über einen Laminierprozess in einer Warmpresse innig mit der ersten Folie verbunden werden.

Da das Messer keine empfindliche Messzelle wie eine Lambdasonde besitzt, kann auf eine Isolierung des Heizers, bei der Lambdasonde bestehend aus gedruckten Aluminiumoxid-Schichten, verzichtet werden, wodurch der Herstellprozessprozess relativ einfach wird.

In einem Ausführungsbeispiel ist die o.g. zweite Grünfolie vor der Abdeckung dergestalt geformt, dass die Anschlusskontakte auf der bedruckten Folie nicht überdeckt werden.

Fig. 6 zeigt eine vereinfachte Darstellung von Formen der Schneide 124 gemäß Ausführungsbeispielen der vorliegenden Erfindung. Bei der Schneide 124 kann es sich um einen Ausschnitt einer Variante einer in Fig. 1, Fig. 2 oder Fig. 5 dargestellten Klinge 100 handeln. Fig. 6 zeigt beispielhaft fünf Varianten von Schneiden 124. Dabei sind, in der Darstellung von links nach rechts, die ersten beiden Schneiden 124 einseitig geschliffen, die mittlere Schneide ist mittig zur Klinge mit geraden Schneidflächen und die beiden letzten Schneiden sind mittig, doppelt geschliffen. Dabei sind die seitlichen Schneidflächen gerade, konvex oder konkav ausgebildet, oder eine Kombination daraus. So weist die letzte Schneide 124 von der Schneidkante ausgehend erst eine konkave Form auf, die dann in eine konvexe Form übergeht.

Die Lage der Grenzfläche zwischen der ersten Keramikschicht und der zweiten Keramikschicht im Verhältnis zur Schneidkante variiert bei unterschiedlichen Ausführungsbeispielen.

Fig. 7 zeigt eine vereinfachte Darstellung eines Schnellverschlusses 760 der Klinge 100 gemäß einem Ausführungsbeispiel der vorliegenden Erfindung. Bei der Klinge 100 kann es sich um ein Ausführungsbeispiel einer in den vorangegangenen Figuren beschriebenen Klinge 100 handeln. Im Schaft 130 einer Klinge 100 sind zwei Schnellverschlüsse 760 angeordnet, um die klinge 100 mit einem Skalpellhalter zu verbinden. Bei dem Schnellverschluss 760 handelt es sich um ein Ausführungsbeispiel einer Koppelungseinrichtung zum mechanischen Koppeln der Klinge 100 mit einem Skalpellhalter.

Fig. 8 zeigt eine vereinfachte Darstellung eines Schneideinstruments 870 mit einer Klinge 100 und einem Skalpellhalter 872 gemäß einem Ausführungsbeispiel der vorliegenden Erfindung. Bei der Klinge 100 kann es sich um eine Variante einer in den vorangegangenen Figuren beschriebenen Klinge 100 handeln. Die Klinge 100 weist eine Schneide 124, einen Klingenrücken 126 sowie einen Schaft 130 auf. Zumindest auf der sichtbaren Klingenseite 128 sind zwei Öffnungen 456 angrenzend an die Schneide 124 angeordnet. Im Bereich des Schafts 130 ist die Klinge 100 mit dem Skalpellhalter 872 mechanisch gekoppelt beziehungsweise verbunden. Der Skalpellhalter 872 weist eine Verriegelungseinrichtung 874, einen Tank 876 sowie einen Energiespeicher 878 auf. Der Tank 876 ist mit einem Fluidkanal der Klinge 100 über einen in dem Skalpellhalter ausgebildeten Fluidkanal verbunden. Der Energiespeicher 878 ist über entsprechende Leitungen mit den Anschlüssen der in der Klinge 100 angeordneten Heizeinrichtung verbunden.

In einem nicht dargestellten Ausführungsbeispiel ist zwischen dem Tank 876 und dem Anschluss des Tanks über einen Fluidkanal zur Klinge 100 eine Pumpe zum Fördern eines Fluids angeordnet.

Anstelle eines Energiespeichers 878 oder einem Tank 876 kann auch eine Schnittstelle zum Anschluss einer externen Fluidleitung oder einer elektrischen Energieversorgung vorgesehen sein.

Der Skalpellhalter 872 weist einen einen Anschluss zum Kontaktieren der Heizeinrichtung (106) der Klinge (100) auf. Weiterhin weist der dargestellte Skalpellhalter einen optionalen Fluidanschluss zur Fluidzuführung und/oder Fluidabführung auf. So kann die Heizeinrichtung der Klinge 100 sowie der Fluidkanal der Klinge 100 einfach kontaktiert werden.

Die Koppelung zwischen der Klinge 100 und dem Skalpellhalter 872 kann in einem Ausführungsbeispiel mit einem adaptierten oder erweiterten System wie dem bekannten Standard-System, Bayha-System, OR-System oder Hohlmeißel-System erfolgen. Dabei sind die Systeme um die Anschlüsse für die Heizeinrichtung und ergänzend oder alternativ für den Fluidkanal zu erweitern.

Je nach Ausführungsbeispiel kann es sich bei dem Schneideinstrument 870 beispielsweise um ein beheizbares Skalpell zur instantanen Koagulation oder ein beheizbares Messer mit integrierten Kanälen für Medienabsaugung oder Zuführung für die Medizintechnik handeln.

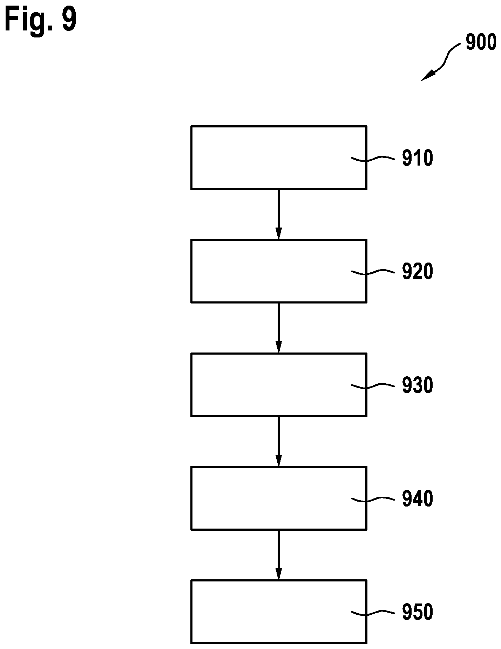

Fig. 9 zeigt ein Ablaufdiagramm eines Verfahrens 900 gemäß einem Ausführungsbeispiel der vorliegenden Erfindung. Das Verfahren 900 zum Herstellen einer Klinge für ein Schneideinstrument umfasst drei Schritte 910, 920, 930 des Anordnens, einen Schritt 940 des Temperierens und einen Schritt 950 des Schleifens.

Im Schritt 910 wird eine erste Grünfolie zum Erzeugen einer ersten Keramikschicht angeordnet, im Schritt 920 wird eine Heizeinrichtung auf der ersten Keramikschicht angeordnet und schließlich im Schritt 930 eine zweite Grünfolie zum Erzeugen einer zweiten Keramikschicht auf der ersten Grünfolie und der Heizeinrichtung angeordnet, sodass die Heizeinrichtung zwischen der ersten Grünfolie und der zweiten Grünfolie angeordnet ist. Darauf folgt im Schritt 940 des Temperierens ein Entbindern und/oder Sintern der Anordnung aus erster und zweiter Grünfolie mit der Heizeinrichtung, wobei die erste Keramikschicht und die zweite Keramikschicht entstehen, die die Heizeinrichtung umschließen. Abschließend wird im Schritt 950 des Schleifens die gesinterte Anordnung geschliffen, um eine Klinge für ein chirurgisches Instrument zu erhalten. Bei der Klinge kann es sich dabei um eine Variante einer in den vorangegangenen Figuren beschriebenen und gezeigten Klinge 100 handeln.

Über einen Fräs-/Bohrprozess können gezielt Kavitäten und Durchgänge in die Grünfolie eingebracht werden. Die zweite Grünfolie trägt durchgängige Kavitäten (beispielsweise Bohrungen) für den Einlass/Auslass von Flüssigkeiten/Medikamenten.

Die kanalartigen Kavitäten können in den Abmessungen von 0,25 - 3mm ausgeführt sein. Sie können kreisrund, wie auch schlitzförmig sein. Denkbar ist auch, dass die Öffnungen schräg durch das Material zum Sammelkanal im Inneren geführt sind, um günstigere Strömungsbedingungen beim Einlass/Auslass und der internen Medienführung einzustellen. Der Sammelkanal kann alternativ auch über einen Siebdruckprozess realisiert werden. Hierfür werden die kanalbildenden Strukturen mit einer rein organischen Dickschichtpaste gedruckt, die beim Entbindern vollständig ausbrennt. Laminiert man beide Grünfolien durch passgenaues Übereinanderlegen und Verpressen in der Warmpresse miteinander und entbindert das Laminat, bleiben Hohlräume an der Stelle zurück, die zuvor mit der rein organischen Paste gedruckt wurde. Auf diesem Weg können sehr schmale und feine Kanalstrukturen dargestellt werden.

Aussparungen für den Kontaktbereich sind im rückwärtigen Teil des Messers angelegt. Analog zu den keramischen Abgassensoren kann im Mehrfachnutzen gedruckt werden, wobei auch hier vor der Sinterung eine Vereinzelung zu separaten Einheiten vorgenommen wird.

Im anschließenden Sinterprozess bei 1250°C - 1385°C für 5h findet die Umwandlung zu einem monolithischen Bauteil statt. Das Gesamtdesign der Klinge kann völlig symmetrisch ausgeführt werden, sodass kein Verzug während der Sinterung zu erwarten ist - möglicherweise kann somit auch auf die aufwendige Einzelbeschwerung der Teile während der Sinterung verzichtet werden.

Nach der Sinterung erfolgt das Schleifen der Klinge über bekannte Hartbearbeitungsverfahren. Die Klinge kann nun in ihrem ungeschliffenen Mittelteil (analog zu Abgassensoren über Dichtelemente aus Steatit in ein rundes Gehäuse) in einen Skalpellhalter eingeclipst, eingesteckt oder auch in ein Gehäuse verpresst werden, wobei die Anschlusskontakte freiliegend bleiben. Der Anschluss des Heizers erfolgt über lösbare Metallklemmen oder Kontaktclips, wobei auch hier die Kontaktiertechnik von Abgassensoren übernommen werden kann.

Vorteilhaft wäre hier ein einfaches Schnell-Stecksystem in einen für den Arzt "medizintechnisch handhabungsfreundlichen" Skalpellhalter, der gleichzeitig die mechanische Schnellfixierung als auch die elektrische Kontaktierung und Bereitstellung von Medienabsaugung und Mediendosierung darstellt.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Klinge (100) für ein Schneideinstrument (870), für einen medizinischen Einsatz, **gekennzeichnet durch** eine erste Keramikschicht (102), eine zweite Keramikschicht (104) und eine Heizeinrichtung (106), die zwischen der ersten Keramikschicht (102) und der zweiten Keramikschicht (104) angeordnet ist, **dadurch gekennzeichnet, dass** zwischen der ersten Keramikschicht (102) und der zweiten Keramikschicht (104) zumindest ein Fluidkanal (346) ausgebildet ist, wobei der Fluidkanal (346) zumindest eine Öffnung (456) aufweist, die ausgeführt ist eine Absaugung von Flüssigkeiten oder ein Einleiten von Spülmedium oder Medikamenten im Bereich der Klinge zu ermöglichen.

2. Klinge (100) gemäß Anspruch 1, bei der die erste Keramikschicht (102) und die zweite Keramikschicht (104) zu einem monolithischen Bauteil verbunden sind.

3. Klinge (100) gemäß einem der vorangegangenen Ansprüche, bei der die Heizeinrichtung (106) zumindest einen Anschlüss (114, 120) aufweist, der insbesondere gegenüber der zweiten Keramikschicht (104) freiliegt.

4. Klinge (100) gemäß einem der vorangegangenen Ansprüche, bei der die erste Keramikschicht (102) und/oder die zweite Keramikschicht (104) zumindest teilweise aus einer keramischen Grünfolie gefertigt ist und/oder Zirkonoxid aufweist.

5. Klinge (100) gemäß einem der vorangegangenen Ansprüche, bei der die Heizeinrichtung (106) eine Temperaturmesseinrichtung umfasst und/oder die Heizeinrichtung (106) einen Heizdraht aufweist, der eine Materialdicke aufweist und/oder ein Material umfasst, um die Klinge (100) mehrfach auf über 500°C aufzuheizen.

6. Klinge (100) gemäß einem der vorangegangenen Ansprüche, wobei die zumindest eine Öffnung (456) als Einlass und/oder Auslass für ein Fluid im Bereich einer Schneide (124) und/oder einer Klingenseite (128) und/oder einem Klingenrücken (126) und/oder einem Schaft (130) der Klinge (100) ausgeführt ist.

7. Klinge gemäß einem der vorangegangenen Ansprüche, bei der ein Querschnitt des Fluidkanals einen Durchmesser in einem Toleranzbereich zwischen 0,25 und 3 mm aufweist.

8. Klinge (100) gemäß einem der vorangegangenen Ansprüche, bei der der Querschnitt des Fluidkanals (346) und/oder die zumindest eine Öffnung (456) zumindest abschnittsweise kreisrund und/oder ellipsoid und/oder schlitzförmig ausgeformt ist.

9. Klinge (100) gemäß einem der vorangegangenen Ansprüche, bei der eine Ebene, in der die Heizeinrichtung (106) angeordnet ist, durch eine Schneide (124) der Klinge (100) verläuft, insbesondere wobei die Klinge (100) symmetrisch zu der Ebene ausgeformt ist, und/oder bei der die Klinge (100) mit einem standardisierten Skalpellhalter (872) kombinierbar ist.

10. Skalpellhalter (872) aufweisend eine Klinge (100) für ein Schneideinstrument (870) gemäß einem der vorangegangenen Ansprüche, wobei der Skalpellhalter (872) zumindest einen Anschluss zum Kontaktieren der Heizeinrichtung (106) der Klinge (100) aufweist.

11. Skalpellhalter (872) gemäß Anspruch 10, mit einem Fluidanschluss zur Fluidzuführung und/oder Fluidabführung für den Fluidkanal (346) der Klinge (100).

12. Verfahren (900) zum Herstellen einer Klinge (100) für ein Schneideinstrument (870), wobei das Verfahren die folgenden Schritte umfasst:
Anordnen (910) einer ersten Keramik-bildenden Lage zum Erzeugen einer ersten Keramikschicht (102);
Anordnen (920) einer Heizeinrichtung (106) auf der ersten Keramik-bildenden Lage und/oder der ersten Keramikschicht (102);
Anordnen (930) einer zweiten Keramik-bildenden Lage zum Erzeugen einer zweiten Keramikschicht (104) auf der ersten Keramik-bildenden Lage, sodass die Heizeinrichtung (106) zwischen der ersten Keramik-bildenden Lage und der zweiten Keramik-bildenden Lage angeordnet ist;
Temperieren (940) der Anordnung aus erster und zweiter Keramik-bildender Lage mit der Heizeinrichtung, wobei die Keramik-bildenden Lagen in Keramikschichten (102, 104) gewandelt werden, wobei die erste Keramikschicht (102) und die zweite Keramikschicht (104) die Heizeinrichtung (106) umschließen; und
Schleifen (950) der gesinterten Anordnung, um die Klinge (100) für das Schneideinstrument (870) und/oder ein chirurgisches Instrument zu erhalten.

## Claims

1. Blade (100) for a cutting instrument (870), for medical use, **characterized by** a first ceramic layer (102), a second ceramic layer (104) and a heating device (106), which is arranged between the first ceramic layer (102) and the second ceramic layer (104), **characterized in that** at least one fluid channel (346) is formed between the first ceramic layer (102) and the second ceramic layer (104), wherein the fluid channel has at least one opening (456), which opening is configured to make possible extraction of fluids by suction or else also introduction of flushing medium or drugs in the region of the blade.

2. Blade (100) according to Claim 1, in the case of which the first ceramic layer (102) and the second ceramic layer (104) are connected to form a monolithic component.

3. Blade (100) according to one of the preceding claims, in the case of which the heating device (106) has at least one connection (114, 120) which is exposed in particular in relation to the second ceramic layer (104).

4. Blade (100) according to one of the preceding claims, in the case of which the first ceramic layer (102) and/or the second ceramic layer (104) are/is produced, at least in part, from a green ceramic film and/or have/has zirconium oxide.

5. Blade (100) according to one of the preceding claims, in the case of which the heating device (106) comprises a temperature-measuring device and/or the heating device (106) has a heating wire, which has a material thickness, and/or comprises a material, for heating up the blade (100) a number of times to above 500°C.

6. Blade (100) according to one of the preceding claims, wherein the at least one opening (456) is configured in the form of an inlet and/or outlet for a fluid in the region of a cutting edge (124) and/or of a blade side (128) and/or a blade spine (126) and/or a stem (130) of the blade (100).

7. Blade according to one of the preceding claims, in the case of which a cross section of the fluid channel has a diameter in a tolerance range between 0.25 and 3 mm.

8. Blade (100) according to one of the preceding claims, in the case of which the cross section of the fluid channel (346) and/or the at least one opening (456) are/is circular and/or ellipsoidal and/or slot-shaped at least in certain regions.

9. Blade (100) according to one of the preceding claims, in the case of which a plane in which the heating device (106) is arranged runs through a cutting edge (124) of the blade (100), in particular wherein the blade (100) is formed symmetrically in relation to the plane, and/or in the case of which the blade (100) can be combined with a standard scalpel holder (872).

10. Scalpel holder (872) having a blade (100) for a cutting instrument (870) according to one of the preceding claims, wherein the scalpel holder (872) has at least one connection for making contact with the heating device (106) of the blade (100).

11. Scalpel holder (872) according to Claim 10, having a fluid connection for fluid-feeding and/or fluid-removal purposes for the fluid channel (346) of the blade (100).

12. Method (900) of producing a blade (100) for a cutting instrument (870), wherein the method comprises the following steps:
arranging in place (910) a first ceramic-forming material layer for generating a first ceramic layer (102);
arranging (920) a heating device (106) on the first ceramic-forming material layer and/or the first ceramic layer (102);
arranging (930) a second ceramic-forming material layer, for generating a second ceramic layer (104), on the first ceramic-forming material layer, and therefore the heating device (106) is arranged between the first ceramic-forming material layer and the second ceramic-forming material layer;
tempering (940) the arrangement made up of the first and second ceramic-forming material layers with the heating device, wherein the ceramic-forming material layers are converted into ceramic layers (102, 104), wherein the first ceramic layer (102) and the second ceramic layer (104) enclose the heating device (106); and
grinding (950) the sintered arrangement to obtain the blade (100) for the cutting instrument (870) and/or a surgical instrument.

## Revendications

1. Lame (100) pour un instrument de coupe (870), destinée à un usage médical, **caractérisée par** une première couche céramique (102), une seconde couche céramique (104) et un dispositif de chauffage (106), qui est disposé entre la première couche céramique (102) et la seconde couche céramique (104), **caractérisée en ce qu'**au moins un canal de fluide (346) est formé entre la première couche céramique (102) et la seconde couche céramique (104), dans laquelle le canal de fluide (346) présente au moins une ouverture (456), qui est réalisée de façon à permettre une aspiration de liquides ou une introduction d'un fluide de rinçage ou d'un médicament dans la région de la lame.

2. Lame (100) selon la revendication 1, dans laquelle la première couche céramique (102) et la seconde couche céramique (104) sont assemblées en un composant monolithique.

3. Lame (100) selon l'une quelconque des revendications précédentes, dans laquelle le dispositif de chauffage (106) présente au moins un raccord (114, 120), qui est libre en particulier en face de la seconde couche céramique (104).

4. Lame (100) selon l'une quelconque des revendications précédentes, dans laquelle la première couche céramique (102) et/ou la seconde couche céramique (104) est fabriquée au moins en partie à partir d'une feuille céramique crue et/ou présente de l'oxyde de zirconium.

5. Lame (100) selon l'une quelconque des revendications précédentes, dans laquelle le dispositif de chauffage (106) comprend un dispositif de mesure de la température et/ou le dispositif de chauffage (106) présente un fil chauffant, qui présente une épaisseur de matière et/ou qui comprend une matière, permettant de chauffer la lame (100) plusieurs fois au-delà de 500°C.

6. Lame (100) selon l'une quelconque des revendications précédentes, dans laquelle ladite au moins une ouverture (456) est réalisée sous la forme d'une entrée et/ou d'une sortie pour un fluide dans la région d'un tranchant (124) et/ou d'un côté de lame (128) et/ou d'un dos de lame (126) et/ou d'une tige (130) de la lame (100).

7. Lame selon l'une quelconque des revendications précédentes, dans laquelle une section transversale du canal de fluide présente un diamètre dans une plage de tolérance entre 0,25 et 3 mm.

8. Lame (100) selon l'une quelconque des revendications précédentes, dans laquelle la section transversale du canal de fluide (346) et/ou ladite au moins une ouverture (456) est réalisée au moins partiellement sous forme circulaire et/ou sous forme ellipsoïdale et/ou sous forme de fente.

9. Lame (100) selon l'une quelconque des revendications précédentes, dans laquelle un plan, dans lequel est disposé le dispositif de chauffage (106), passe par un tranchant (124) de la lame (100), en particulier dans laquelle la lame (100) est formée de façon symétrique par rapport au plan, et/ou dans laquelle la lame (100) peut être combinée avec un porte-scalpel normalisé (872).

10. Porte-scalpel (872), présentant une lame (100) pour un instrument de coupe (870) selon l'une quelconque des revendications précédentes, dans lequel le porte-scalpel (872) présente au moins un raccord pour la mise en contact du dispositif de chauffage (106) de la lame (100).

11. Porte-scalpel (872) selon la revendication 10, avec un raccord de fluide pour l'arrivée de fluide et/ou l'évacuation de fluide pour le canal de fluide (346) de la lame (100).

12. Procédé (900) de fabrication d'une lame (100) pour un instrument de coupe (870), dans lequel le procédé comprend les étapes suivantes:
disposer (910) une première couche génératrice de céramique pour produire une première couche céramique (102) ;
disposer (920) un dispositif de chauffage (106) sur la première couche génératrice de céramique et/ou sur la première couche céramique (102);
disposer (930) une seconde couche génératrice de céramique pour produire une seconde couche céramique (104) sur la première couche génératrice de céramique, de telle manière que le dispositif de chauffage (106) soit disposé entre la première couche génératrice de céramique et la seconde couche génératrice de céramique;
réguler (940) la température de l'agencement des première et seconde couches génératrices de céramique avec le dispositif de chauffage, dans lequel on convertit les couches génératrices de céramique en couches céramiques (102, 104), dans lequel la première couche céramique (102) et la seconde couche céramique (104) enferment le dispositif de chauffage (106); et
meuler (950) l'agencement fritté, afin d'obtenir la lame (100) pour l'instrument de coupe (870) et/ou un instrument chirurgical.
